(19) **Europäisches Patentamt / European Patent Office / Office européen des brevets**

(11) **EP 4 606 892 A1**

(12) # EUROPEAN PATENT APPLICATION

(43) Date of publication:
**27.08.2025 Bulletin 2025/35**

(21) Application number: **24159462.1**

(22) Date of filing: **23.02.2024**

(51) International Patent Classification (IPC):
**C12N 7/02** *(2006.01)* **C12N 15/86** *(2006.01)*
**C12N 15/864** *(2006.01)*

(52) Cooperative Patent Classification (CPC):
**C12N 15/86; C12N 7/00;** C12N 2750/14143;
C12N 2750/14151

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Merck Patent GmbH**
**64293 Darmstadt (DE)**

(72) Inventor: **The designation of the inventor has not yet been filed**

(74) Representative: **Merck Patent Association**
**Merck Patent GmbH**
**64271 Darmstadt (DE)**

(54) **METHODS FOR THE PRODUCTION OF VIRAL PARTICLES**

(57)     The present invention relates to methods for improving the production of viral particles, like adeno-associated viral particles, especially to methods for reducing aggregation of viral particles by removing at least divalent cations, e.g. by treating the particles with a chelator.

EP 4 606 892 A1

**Description**

The present invention relates to methods for improving the production of viral particles, like adeno-associated viral particles, especially to methods for reducing aggregation of viral particles by removing at least divalent cations, favorably by treating the particles with a chelator.

Large-scale manufacturing for clinical grade viral gene therapy vectors faces numerous obstacles including scalable unit operations for vector production and purification that removes contaminants while maintaining critical parameters like vector stability, titer and transduction efficiency.

One parameter that can affect purification and also transduction efficiency of viral particles is viral aggregation. Viral aggregation is a complex phenomenon that affects many viral families. Several approaches have been made to influence and especially reduce viral aggregation. In WO 2005/118792 it is suggested to reduce aggregation of AAV particles with a combination of high ionic strength and modest osmolarity. CN113980917 discloses a lentivirus dissolution buffer comprising the detergent Tween® 80 for reducing viral aggregation.

Nevertheless, viral vector production still lacks the possibility to achieve high titers of genome containing and active viral particles in short processing times.

It has now been found that incubating the sample comprising viral particles, e.g. after cell lysis, with a chelator that is able to bind and trap at least divalent cations and then isolating or purifying the viral particles from the sample effectively reduces aggregation of the resulting viral particles

The present invention is thus directed to a method for reducing aggregation of viral particles by

a) Providing a sample comprising viral particles and process related impurities, especially host cell proteins and/or host cell DNA
b) Reducing the amount of divalent cations in the sample, favorably by treating the sample with a chelator
c) Separating the viral particles from process related impurities

In a preferred embodiment the chelator is EDTA.

In a preferred embodiment step b) is performed by incubating the sample with the chelator for at least 30 seconds, preferably for 1 minute to 10 hours.

In another preferred embodiment the viral particles are AAV particles.

In another preferred embodiment the sample is a clarified sample, for example a clarified lysate.

In another preferred embodiment the method further comprises treating the sample with a nuclease, whereby the nuclease is added prior, in parallel or after the addition of the chelator.

In a preferred embodiment, step c) comprises a filtration and/or a chromatography step. In one embodiment, the sample is first filtered and then subjected to a chromatographic separation.

In a very preferred embodiment step c) comprises an affinity chromatography step. Preferably, the sample of step b) is optionally filtered and then fed onto the affinity matrix.

The present invention is also directed to a method of reducing and/or preventing aggregation of viral particles in a sample comprising adding one or more chelators to the sample whereby the amount of chelators is sufficient to bind and/or trap all divalent and optionally also all trivalent cations present in the sample.

The present invention is also directed to a process for producing viral particles comprising the steps of

a) Culturing cells to produce viral particles
b) Optionally lysing cells which comprise said viral particles to release the viral particles
c) Treating the viral particles released by the cells with a chelator
d) Performing at least one chromatographic purification

*In a preferred embodiment the sample obtained from the a) or b) is clarified prior to step c)*

In one embodiment the sample obtained from step c) is filtered using a filter with a pore size between 0.1 and 0.8 $\mu$m, preferably between 0.2 and 0.8 $\mu$m.

In a preferred embodiment, the chelator is EDTA.

In another preferred embodiment, the viral particles are AAV particles.

In one embodiment, the method of the present invention comprises one or more of the following steps:

-    clarification
-    filtration
-    dialysis/diafiltration
-    tangential flow filtration
-    treatment with nuclease, e.g. RNase and/or DNase

- treatment with chloroform
- ion exchange chromatography
- multimodal chromatography
- affinity chromatography
- hydrophobic interaction chromatography
- centrifugation
- PEG precipitation
- flocculation

The present invention is also directed to a composition comprising viral particles, a pH buffer and a chelator. Such viral particles may result from the process of the present invention and be substantially free of process related impurities. The composition may also still comprise process related impurities, especially host cell proteins and host cell DNA. Storage of the particles as a composition comprising a chelator and a suitable pH buffer ensures that aggregation is reduced and the particles can be subjected to any further treatment as suitable. The composition can for example be subjected to freezing in liquid nitrogen.

Figures 1 and 2 show the pressure drop with and without EDTA pre-treatment. Fig. 1 shows the pressure curve without EDTA pre-treatment. Fig. 2 shows the pressure curve with EDTA pre-treatment. Details can be found in Example 2.2.

Fig. 3 shows a DLS analysis of elution pool fractions with and without EDTA pre-treatment. Details can be found in Example 2.1.

Before describing the present invention in detail, it is to be understood that this invention is not limited to specific compositions or process steps, as such may vary. It must be noted that, as used in this specification and the appended claims, the singular form "a", "an" and "the" include plural referents unless the context clearly dictates otherwise. Thus, for example, reference to "a ligand" includes a plurality of ligands and reference to "an antibody" includes a plurality of antibodies and the like.

Unless defined otherwise, all technical and scientific terms used herein have the same meaning as commonly understood by one of ordinary skill in the art to which this invention is related. The following terms are defined for purposes of the invention as described herein.

The term "viral vector" or "viral particle" is widely used to refer to a viral particle that mediates nucleic acid transfer. Viral particles include various viral components and sometimes also host cell components in addition to nucleic acid(s). Illustrative viral vectors include, but are not limited to, retrovirus vectors, lentivirus vectors, adenovirus vectors, adeno-associated virus (AAV) vectors, vaccinia virus vectors, and herpes simplex virus (HSV) vectors. Preferred viral vectors are lentivirus vectors and AAV vectors, most preferred are AAV vectors.

Lentiviral vectors are part of a larger group of retroviral vectors. A detailed list of lentiviruses may be found in Coffin et al. (1997) "Retroviruses" Cold Spring Harbor Laboratory Press Eds: JM Coffin, SM Hughes, HE Varmus pp 758-763). In brief, lentiviruses can be divided into primate and non-primate groups. Examples of primate lentiviruses include but are not limited to: the human immunodeficiency virus (HIV), the causative agent of human auto-immunodeficiency syndrome (AIDS), and the simian immunodeficiency virus (SFV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

The best-known lentivirus is the Human Immunodeficiency Virus (HIV), which causes AIDS. The lentiviral vector is used because it is safe (after disabling the HIV genes), low cellular immune response, and can undergo transduction (gene modification) of both dividing and non-dividing cells. The FDA has approved the use of lentiviral vectors in cancer therapy.

Adeno-associated virus (AAV) have been characterized and developed as a potent viral vector to deliver genes in vitro in cultured cells and also in vivo. AAV is meanwhile a leading platform for in vivo delivery of gene therapies. Adeno-associated virus (AAV) is a member of the Parvoviridae family. The AAV genome is composed of a linear single-stranded DNA molecule which contains approximately 4.7 kilobases (kb) and consists of two major open reading frames encoding the non-structural Rep (replication) and structural Cap (capsid) proteins. Flanking the AAV coding regions are two cis-acting inverted terminal repeat (ITR) sequences, approximately 145 nucleotides in length, with interrupted palindromic sequences that can fold into hairpin structures that function as primers during initiation of DNA replication. In addition to their role in DNA replication, the ITR sequences have been shown to be necessary for viral integration, rescue from the host genome, and encapsidation of viral nucleic acid into mature virions (Muzyczka, (1992) Curr. Top. Micro. Immunol. 158:97-129).

Multiple serotypes of AAV exist and offer varied tissue tropism. Known serotypes include, for example, AAV1, AAV2, AAV3, AAV4, AAV5, AAV6, AAV7, AAV8, AAV9, AAV10 and AAV11.

Vectors derived from AAV are particularly attractive for delivering genetic material because they are able to infect (transduce) a wide variety of non-dividing and dividing cell types including muscle fibers and neurons and they are devoid of the virus structural genes, thereby eliminating the natural host cell responses to virus infection, e.g., interferon-mediated responses. In addition, wild-type viruses have never been associated with any pathology in humans.

According to the present invention scAAV are also within the group of AAVs. Self-complementary adeno-associated vectors (scAAV) are viral vectors engineered from the naturally occurring adeno-associated virus (AAV) for use in gene therapy. ScAAV is termed "self-complementary" because the coding region has been designed to form an intramolecular double-stranded DNA template.

Thus, in some embodiments, by an "AAV" is meant a vector or virus derived from an adeno-associated virus serotype, including without limitation, AAV-1, AAV-2, AAV-3, AAV-4, AAV -5, AAV- 6, AAV-7, AAV -8, AAV-9, AAV-10 and AAV-11. AAV vectors can have one or more of the AAV wild-type genes deleted in whole or part, e.g., the rep and/or cap genes, but retain functional flanking ITR sequences. Functional ITR sequences are necessary for the rescue, replication and packaging of the AAV virion. Thus, an AAV vector is defined herein to include at least those sequences that provide for replication and packaging (e.g., functional ITRs) of the virus. The ITRs need not be the wild-type nucleotide sequences, and may be altered, e.g., by the insertion, deletion or substitution of nucleotides, so long as the sequences provide for functional rescue, replication and packaging. In one embodiment, the vector is an AAV-9 vector, with AAV-2 derived ITRs. Also by an "AAV" is meant the protein shell or capsid, which provides an efficient vehicle for delivery of vector nucleic acid to the nucleus of target cells.

The term "AAV" as used herein is intended to also encompass recombinant AAV.

Adeno-associated virus (AAV) are herein also called viruses, viral particles or viral vectors.

Adeno-associated virus (AAV) have been characterized and developed as a potent viral vector to deliver genes in vitro in cultured cells and also in vivo. AAV is meanwhile a leading platform for in vivo delivery of gene therapies. AAV is a small, non-enveloped virus containing a single-stranded DNA genome of approximately 4.7 kb, consisting of two inverted terminal repeats (ITRs) that are capable of forming T-shape secondary structure and acting as origins of genome replication, one rep region that encodes four overlapping replication proteins, Rep78, Rep68, Rep52, and Rep40, and one cap region that encodes three structural proteins, VP1, VP2, and VP3, and an assembly activating protein (AAP). Naturally isolated serotypes 1-9 of the AAV viruses share the genomic structure although these serotypes may display different tissue tropism. As the AAVs seem to be nonpathogenic, show an efficient transduction and a stable expression, they are regarded as being one of the most promising gene delivery vehicles.

Transduction efficiency defines how effectively a vector can deliver genetic material into cells. It can be described by transducing units. Transducing units (TU) are the number of functional viral particles in a solution that are capable of transducing a cell and expressing the transgene. The higher the number of transducing units in a sample the higher is the transduction efficiency of the sample.

Consequently, in the context of viral vector manufacturing, the most common manufacturability measures include viral titer (as measured by VP/ml, VP = number of total viral vectors); and the transducing unit titer (as measured by TU/ml); a related feature is transduction efficiency, or the TU/VP ratio, which indicates how many functional viral vectors are contained out of the total number of viral vectors. Such titers are typically quantified with cell transduction assays (e g. fluorometric microscopy on transduced cells like HeLa cells or FACS analysis). Transduction efficiency can be measured according to the method described in the Examples.

As used herein, the term "cell" or "cell line" refers to a single cell or to a population of cells capable of continuous or prolonged growth and division in vitro. In some embodiments, e.g. the terms "HEK293 cells", "293 cells" or their grammatical equivalents are used interchangeably here and refer to the host/packing cell line used in the methods disclosed herein.

Suitable cells and cell lines have been described for use in production of viral vectors like lentiviral vectors, AAVs and AdVs. The cells themselves may be selected from any biological organism, including prokaryotic (e.g., bacterial) cells and eukaryotic cells including insect cells, yeast cells and mammalian cells. Particularly desirable host cells are selected from among any mammalian species, including, without limitation, A549, WEHI, 3T3, 10T1/2, BHK, MDCK, COS 1, COS 7, BSC 1, BSC 40, BMT 10, VERO, WI38, HeLa, a HEK293 cell, Saos, C2C12, L cells, HT1080, HepG2 and primary fibroblast, hepatocyte and myoblast cells derived from mammals including human, monkey, mouse, rat, rabbit, and hamster.

During the manufacturing of especially AAVs, a percentage of capsids might not incorporate any of the transgenes and are referred to as empty capsids, empty AAVs or empty AAV particles. Additionally, capsids that contain fragments of the transgene or process related impurities are called partial capsids, partial AAVs or partial AAV particles. These undesired impurities are co-produced with the full capsids or full AAVs which contain the full length of the desired transgene.

Purification means to increase the degree of purity of a target molecule, in this case the viral particles like AAVs, e.g. by removing one or more impurities.

The term "impurity" or "contaminant" as used herein, refers to any foreign or objectionable molecules or species, including a biological macromolecule such as DNA, RNA, one or more host cell proteins, nucleic acids, endotoxins, lipids, impurities of synthetic origin like detergents, partial and/or empty viral particles as well as one or more additives which may be present in a sample containing the viral particles to be purified and thus to be separated from one or more of the impurities. Viral particle impurities include both, process-related impurities and/or product-related impurities. Exemplary process-related impurities include, but are not limited to, residual host-cell components (e,g., proteins, DNA - including extra-viral, chromatin-associated DNA - and/or RNA), residual viral production components (e.g., plasmid DNA, and/or helper viruses), residual cell culture components (e.g., antibiotics, supplements, inducers, and/or growth factors), and residual purification components (e.g. buffers, inorganic salts, enzymes, media, and/or detergents), as well as other contaminants. The aim of purifying viral particles is typically to generate viral particles that are substantially free of process related impurities like a host-cell protein, a host-cell DNA, a host-cell RNA, or a combination thereof. Exemplary product-related impurities can include, but are not limited to, empty capsids (where undesirable), aggregated viral particles, and degraded viral particles.

It is understood that a sample can be "substantially free" of one or more impurities, but continue to have a small amount (e,g, undetectable level, or below an acceptable range) of one or more impurities, and that "substantially free" does not require complete removal of all impurities.

As used herein, and unless stated otherwise, the term "sample" refers to any composition or mixture that contains viral particles. Samples may be derived from biological or other sources. Biological sources include eukaryotic and prokaryotic sources, such as plant and animal cells, tissues and organs. The sample may also include diluents, buffers, detergents, and contaminating species, debris and the like that are found mixed with the target molecule. The sample may be "partially purified" (i.e., having been subjected to one or more purification steps, such as filtration steps) or may be obtained directly from a host cell producing the viral particles, e.g., the sample may comprise harvested cell culture fluid.

The terms "purifying," "separating," or "isolating," as used interchangeably herein, refer to increasing the degree of purity of the target viral particles from a composition or sample comprising the target viral particles and one or more impurities.

The term "chromatography" refers to any kind of technique which separates an analyte of interest (e.g. a target viral particle) from other molecules present in a sample. Usually, the target viral particle is separated from other molecules as a result of differences in rates at which the individual molecules of the mixture bind to and/or migrate through a chromatography matrix under the influence of a moving phase.

The term "matrix" or "chromatography matrix" are used interchangeably herein and refers to a solid phase through which the sample migrates in the course of a chromatographic separation. The matrix typically comprises a base material and ligands covalently bound to the base material. The matrix of the present invention comprises or consists e.g. of particles, a membrane or a monolith, preferably the base material is a membrane.

A "ligand" is a functional group that is part of the chromatography matrix, typically it is attached to the base material of the matrix, and that determines the binding properties and interaction properties of the matrix. Examples of "ligands" include, but are not limited to, ion exchange groups, hydrophobic interaction groups, hydrophilic interaction groups, thiophilic interactions groups, metal affinity groups, affinity groups, bioaffinity groups, and mixed mode groups (combinations of the aforementioned). It is also possible that one ligand has more than one binding / interaction property.

The ligands may be attached to the base material of the matrix by any type of covalent attachment. Covalent attachment can for example be performed by directly bonding the functional groups to suitable residues on the base material like OH, NH2, carboxyl, phenol, anhydride, aldehyde, epoxide or thiol etc. It is also possible to attach the ligands via suitable linkers. It is also possible to generate the matrix by polymerizing monomers comprising the ligands and a polymerizable moiety. Examples of matrices generated by polymerization of suitable monomers are polystyrene, polymethacrylamide or polyacrylamide based matrices generated by polymerizing suitable styrole or acryloyl monomers.

In another embodiment the chromatography matrix can be generated by grafting the ligands onto the base material or from the base material. For grafting from processes with controlled free-radical polymerisation, such as, for example, the method of atom-transfer free-radical polymerisation (ATRP), are suitable. A very preferred one-step grafting from polymerisation reaction of acrylamides, methacrylates, acrylates, methacrylates etc. which are functionalized e.g. with ionic, hydrophilic or hydrophobic groups can be initiated by cerium(IV) on a hydroxyl-containing support, without the support having to be activated.

When the chromatography matrix is used in a chromatographic separation it is typically used in a separation device, also called housing, as a means for holding the matrix. Suitable housing are known to the skilled person.

In certain embodiments, especially if the chromatography matrix is a membrane, the chromatography matrix is arranged in the housing in a substantially coplanar stack of substantially coextensive sheets, a substantially tubular configuration, or a substantially spiral wound configuration.

A "buffer" is a solution that resists changes in pH by the action of its acid-base conjugate components. Various buffers which can be employed depending, for example, on the desired pH of the buffer are described in Buffers. A Guide for the Preparation and Use of Buffers in Biological Systems, Gueffroy, D., ed. Calbiochem Corporation (1975). Non-limiting examples of buffers include MES, MOPS, MOPSO, Tris, HEPES, phosphate, acetate, citrate, succinate, and ammonium buffers, as well as combinations of these.

According to the present invention the term "buffer" or "solvent" is used for any liquid composition that is used to load, wash, elute, re-equilibrate, strip and/or sanitize a chromatography matrix.

A membrane as chromatographic matrix can be distinguished from particle-based chromatography by the fact that the interaction between a solute, e.g. the target AAVs or contaminants, and the matrix does not take place in the dead-ended pores of a particle, but mainly in the throughpores of the membrane. Exemplary types of membranes are flat sheet systems, stacks of membranes, microporous polymer sheets with incorporated cellulose, polystyrene or silica-based membranes as well as radial flow cartridges, hollow fiber modules and hydrogel membranes. Preferred are hydrogel membranes. Such membranes comprise a membrane support and a hydrogel formed within the pores of said support. The membrane support provides mechanical strength to the hydrogel. The hydrogel determines the properties of the final product, like pore size and binding chemistry.

The membrane support can consist of any porous membrane like polymeric membranes, ceramic based membranes and woven or non-woven fibrous material. Suitable polymeric materials for membrane supports are cellulose or cellulose derivatives as well as other preferably inert polymers like polyethylene, polypropylene, polybutylenterephthalate or polyvinylidene-difluoride.

The hydrogels can be formed through in-situ reaction of one or more polymerizable monomers with one or more crosslinkers and/or one or more cross-linkable polymers to form a cross-linked gel that has preferably macropores. Suitable polymerizable monomers include monomers containing vinyl or acryl groups. Preferred are monomers comprising an additional functional group that either directly forms the ligand of the matrix or is suitable for attaching the ligands. Suitable crosslinkers are compounds containing at least two vinyl or acryl groups. Further details about suitable membrane supports, monomers, crosslinkers etc. as well as suitable production conditions can be found in WO04073843 and WO2010/027955. Especially preferred are membranes made of an inert, flexible fiber web support comprising assembly within and around the fiber web support a porous polyacrylamide hydrogel with cation exchange as well as hydrophobic groups, like Natrix® type membranes, Merck KGaA, Germany. Further details about suitable hydrogel mixed mode membranes can be found in WO2014018635.

Examples of suitable membranes to be used in the method of the present invention are

- Membranes with a polyethersulfone (PES)-based support and a cross-linked polymeric coating, functionalized with suitable ligands, like Mustang® type membranes, Pall.
- Membranes made of stabilized reinforced cellulose, functionalized with suitable ligands, like Sartobind® type membranes, Sartorius.
- Membranes made of stabilized reinforced cellulose, comprising a hydrogel with suitable ligands, like Sartobind® Jumbo Membranes, Sartorius, made of stabilized reinforced cellulose
- Membranes made of a fine fiber non-woven scaffold comprising a hydrogel with suitable ligands, like 3M™ Emphaze™ Hybrid Purifier type membranes, 3M.
- Membranes made of an inert, flexible fiber web support comprising within and around the fiber web support a porous polyacrylamide hydrogel with suitable cation exchange and hydrophobic ligands, like Natrix® type Chromatography membranes, Merck KGaA, Germany.

A monolith or a monolithic sorbent, similar to a membrane, has throughpores, like interconnected channels, so that liquid can flow from one side of the monolith, through the monolith, to the other side of the monolith. Since the mobile phase is flowing through these throughpores, molecules to be separated are transported by convection rather than by diffusion. Due to their structure monolithic sorbents show flow rate independent separation efficiency and dynamic capacity.

The monolith is typically formed in situ from reactant solutions and can have any shape or confined geometry, typically with frit-free construction, which guarantees convenience of operation. Preferably, monolithic materials have a binary porous structure, mesopores and macropores. The micron-sized macropores are the throughpores and ensure fast dynamic transport and low backpressure in applications; mesopores contribute to sufficient surface area and thus high loading capacity.

The monoliths can be made of organic, inorganic or organic/inorganic hybrid materials. Preferred are organic polymer based monoliths.

The synthesis of organic polymer monoliths is typically done by a one-step polymerization providing a tunable porous structure with tailored functional groups. Generally, a pre-polymerization mixture consisting of the monomers, crosslinkers, porogenic solvents, and initiators in an appropriate ratio is polymerized in a suitable container, also called mould, determining the format of the monolith. Polymerization is typically initiated by heating, use of UV radiation, microwave or γ-ray radiation in the presence of initiators. After reaction for the prescribed time at an appropriate temperature, the resulting material is typically washed with solvents to remove unreacted components and porogenic solvents.

Suitable organic polymers are polymethacrylates, polyacrylamides, polystyrenes, polyurethanes, etc., like Poly(methacrylic acid-ethylene dimethacrylate), Poly(glycidyl methacrylate-ethylene dimethacrylate) or Poly(acrylamide-vinylpyridine-N,N'-methylene bisacrylamide).

Inorganic monoliths can be made of silica or other inorganic oxides. Preferably they are made of silica. Silica monoliths are normally prepared via a sol-gel method with phase separation. This mainly includes hydrolysis, condensation, and polycondensation of silica precursors. Typically, tetraethoxysilane (TEOS) or tetramethylorthosilicate (TMOS) is distributed in a suitable solvent in the presence of a porogen (e.g. polyethylene glycol) (PEG)), followed by the addition of a catalyst, acid or base, or a binary catalyst, acid and base in sequence. After reaction for a prescribed time, the resulting gel-like product is washed with solvents to remove unreacted precursor, porogen, and catalyst, followed by the proper post treatment, typically a heat treatment.

The monoliths can be modified with suitable functional groups, in the present case cation exchange groups and hydrophobic interaction groups, to generate the targeted interaction with the sample comprising the target molecule and thus the targeted separation.

Typically the monoliths are contained in a housing like a column.

Membranes and monoliths can also be produced by 3D printing processes.

Particulate base materials can be prepared, for example, from organic polymers. Organic polymers of this type can be polysaccharides, such as agarose, dextranes, starch, cellulose, etc., or synthetic polymers, such as poly(acrylamides), poly(methacrylamides), poly(acrylates), poly(methacrylates), hydrophilically substituted poly(alkyl allyl ethers), hydrophilically substituted poly(alkyl vinyl ethers), poly(vinyl alcohols), poly(styrenes) and copolymers of the corresponding monomers. These organic polymers can preferably also be employed in the form of a crosslinked hydrophilic network. This also includes polymers made from styrene and divinylbenzene, which can preferably be employed, like other hydrophobic polymers, in a hydrophilized form.

Alternatively, inorganic materials, such as silica, zirconium oxide, titanium dioxide, aluminium oxide, etc., can be employed as particulate base materials. It is equally possible to employ composite materials, i.e., for example, particles which can themselves be magnetised by copolymerisation of magnetisable particles or of a magnetisable core. It is also possible to use core shell materials whereby the shell, i.e. at least the surface or a coating, has OH groups.

However, preference is given to the use of hydrophilic base materials which are stable to hydrolysis or can only be hydrolysed with difficulty since the materials according to the invention should preferably withstand alkaline cleaning or regeneration at e.g. basic pH over an extended use duration. The base matrix may consist of irregularly shaped or spherical particles, whose particle size can be between 2 and 1000 μm. Preference is given to average particle sizes between 3 and 300 μm, in a most preferred embodiment the average particle size is between 20 - 63 μm.

The particulate base material may, in particular, be in the form of nonporous or preferably porous particles. The average pore sizes can be between 2 and 300 nm. Preference is given to pore sizes between 5 and 200 nm, most preferred average pore size is between 40 - 110 nm.

In a very preferred embodiment, the particulate base material is formed by copolymerisation of a hydrophilically substituted alkyl vinyl ether selected from the group of 1,4-butanediol monovinyl ether, 1,5-pentanediol monovinyl ether, diethylene glycol monovinyl ether or cyclo¬hexane¬dimethanol monovinyl ether and divinylethyleneurea (1,3-divinylimidazolin-2-one) as crosslinking agent.

An example of a suitable commercially available vinylether based base material is Eshmuno®, Merck KGaA, Germany.

The base material may equally also be in the form of fibres, hollow fibres or coatings.

The present invention provides methods of preparing and purifying viral vectors, especially for reducing aggregation of viral vectors. It has been found that reducing the aggregation of viral vectors with the method of the present invention leads to several positive effects, like facilitation of certain purification steps like filtration and especially chromatographic purification. The presence of aggregates, like aggregates of viral vectors or aggregates or residues of host cell proteins and/or host cell DNA can negatively influence a filtration process. It can also negatively influence a chromatography process by e.g. clogging of the column. It has also been found that overall the transduction efficiency is improved if divalent cations are reduced or favorably substantially removed from the sample according to the method of the present invention, preferably by treating the sample with a chelator.

Viral vector production is known to the person skilled in the art. In the following, the production of AAV vectors is described in more detail as an example.

AAV vectors can be produced in various cell lines in adherent or suspension cell culture formats using transient transfection or co-infection methods. Preferably, AAV vectors are produced in suspension cell lines. Depending on specific serotypes and production times, viral particles including full, partial and empty species can be secreted out of cells into culture medium or contained inside cells at various ratios.

Initially, stable AAV producer cells were generated by transfection and selection of human-derived cells, like HeLa or HEK293 cells, with an rAAV transfer vector containing the ITR cassette and a packaging construct containing Rep and Cap. Production of recombinant AAV vectors (rAAV) was then achieved by infection with auxiliary viruses such as adenoviruses (AdV) that provide the helper function. After identification of AdV genes required for AAV vector packaging, a helper virus-free method was established using a duo or triple transfection protocol consisting of two or three plasmids including a constructed helper plasmid instead of an auxiliary virus. This system is widely used in research and drug development. In addition, development of baculovirus expression vectors provides another method to produce rAAV viruses in insect Sf9 cells. These different technologies are shown to be able to produce sufficient quantities of rAAV viruses for use in laboratories and clinical trials.

A cell lysis step is generally required at harvest to release viral particles into the supernatant if the viral particles are not secreted by the cells. Suitable methods are known to the experts. For this application, typical cell lysis reagents such as Triton X-100, Tween 20, and NaCl are suitable.

After cell lysis or secretion by the cells, the AAVs need to be purified. Typical AAV purification processes include clarification, concentration and diafiltration using tangential flow filtration, chromatography purification by using affinity chromatography and ion exchange chromatography. In some processes, ultracentrifugation and gradient ultracentrifugation are used instead of chromatography or in addition to chromatography. Final steps in AAV purification typically involve concentration and diafiltration into suitable excipient buffer composition and sterile filtration.

In the following an exemplary process is described in more detail. Typically, the selected cells are expanded in suitable culture media in a bioreactor under suitable conditions. Typically the cells are grown as suspension culture. For example, in suspension culture of HEK293 cells suitable seeding numbers before transfection are 0.5 to 1.1 e6 viable cells per ml.

Suitable methods for the transduction are known in the art. In one embodiment, cells can be transduced in vitro by combining a rAAV with the cells, e.g., in appropriate media, and screening for those cells harboring the DNA of interest using conventional techniques such as Southern blots and/or PCR, or by using selectable markers.

Transfection can be performed using any of the techniques known in the art, including but not limited to electroporation, lipofection, e.g. with a lipofectamine, cationic polymers and cationic lipids. Any suitable transfection media may be used. In one embodiment of the transfection process, adherent or suspension human embryonic kidney (HEK293) cells are transfected with a dual or triple DNA plasmid polyethylenimine (PEI) coprecipitation.

After a suitable virus production period post transfection or infection, the cells are optionally lysed and the viral particles, like AAVs or any other viral particles that have been produced, are harvested. In some embodiments, the cells are dissociated from the bioreactor before the cell lysis process is initiated. In some embodiments, the cells are lysed in situ.

The released viral particles can then be isolated and/or purified, whereby preferably a chromatographic purification on an affinity matrix is included.

Preferably, the mixture obtained from the cell culture or after lysis is first filtered or centrifuged.

In one embodiment the mixture is filtered through a filter that removes large molecule contaminants and cellular debris but that permits viral particles to pass therethrough.

In a preferred embodiment, the released viral particles can be separated and purified from the cell culture medium using clarification. Clarification is a process, which includes at least one filtration step. It might involve one or more steps selected from centrifugation, tangential flow filtration, depth filtration, and sterile filtration. Clarification is a process in which relatively larger components such as cells, lysed cells, cellular debris, protein aggregates and other bulky mainly process related impurities are removed from a solution, typically the solution resulting from culturing cells and optionally lysing them. Clarification filters include depth filtration, charged depth filtration and similar microfiltration techniques. The resulting sample is a clarified sample.

Tangential flow filtration can be used to concentrate the mixture of purified viral particles and to remove salts and process related contaminants like proteins or nucleic acids. Tangential flow filtration (TFF) refers to a generally rapid and efficient method for filtration or purification of a solution containing target product and/or impurities during which a solution or liquid stream flows parallel to a filtering membrane.

Centrifugation can for example be a low speed centrifugation to remove larger particles like cellular debris. This can be for example done at 10000 to 12000 g for 10 to 30 minutes. The released viral particles can be found in the supernatant.

In some embodiments, a nuclease, typically an endonuclease, is added, e.g. to reduce the amount of host cell DNA. It can be added directly to the mixture in the bioreactor before, while or after lysis. The nuclease may be one that degrades both DNA and RNA. In one embodiment, the endonuclease is a genetically engineered endonuclease from Serratia marcescens that is sold under the name Benzonase(R) (EMD Millipore, US). If the activity of the nuclease depends on the presence of certain divalent cations it might be favorable to do the nuclease treatment separately, e.g. prior to the addition of a chelator.

Chromatographic purification of viral particles is known to the skilled person. Chromatographic purification can be performed in bind-elute or flow through mode. When doing affinity chromatography, the target viral particle is bound to the affinity chromatography matrix while the impurities do not bind and flow through. After optional washing of the matrix bound viral particles, the particles are eluted from the matrix with a suitable elution buffer.

The isolation and/or purification of the viral particles typically includes one or more of the following process steps:

- clarification
- filtration
- dialysis/diafiltration
- tangential flow filtration
- treatment with nuclease, e.g. RNase and/or DNase
- treatment with chloroform
- ion exchange chromatography
- affinity chromatography
- multimodal chromatography
- hydrophobic interaction chromatography
- centrifugation
- PEG precipitation
- flocculation

It has been found that treating a sample comprising viral particles and process related impurities with a chelator and subjecting this sample to at least one purification step for separating the viral particles from process related impurities results in a reduction of aggregation of viral particles. Exemplary suitable purification steps are a chromatography step, preferably affinity chromatography or a filtration step, preferably TFF. It has further been found that a sample that has been treated with a chelator and has subsequently been purified comprises less viral aggregates compared to a sample that has not been pre-treated with a chelator and has been subjected to the otherwise identical treatment and purification.

While not wishing to be bound by theories, it is assumed that the chelator reduces the binding of host cell proteins and host cell DNA with the viral particles. One theory of viral aggregation is that said aggregation is supported by host cell proteins and host cell DNA that convey and reinforce binding between themselves and the viral particles. Such binding might be caused by metal cation mediated association of histidine residues on proteins with e.g. nucleic acids. Through chelation of metal cations by a chelator the complex of histidine residues with other histidine residues or nucleic acids is dissociated. The purification step afterwards removes the nucleic acids and proteins from the viral particles so that the compounds cannot re-associate again once the chelator is removed. The purified sample treated with the method of the present invention can even be contacted with divalent cations afterwards without causing an increase in aggregation.

The present invention thus relates to a method for reducing aggregation of viral particles by

a) Providing a sample comprising viral particles and process-related impurities, especially host cell DNA and host cell proteins, more precise at least host cell DNA and/or fragments thereof
b) Reducing or preferably substantially removing divalent cations in the sample, preferably by treating the sample with a chelator
c) Separating the viral particles from process-related impurities, e.g. by one or more steps selected from chromatography, filtration, and flocculation combined with centrifugation or filtration

The process related impurities in step a) are especially host cell proteins and/or host cell DNA, especially host cell DNA or fragments thereof. The sample might be directly taken after cell lysis or the sample may have already been purified, e.g. by one or more centrifugation steps, one or more filtration steps and/or one or more chromatography steps. The sample might have also been treated with a nuclease.

In a preferred embodiment, the sample has not been treated with any chromatography step.

Most preferred the sample is a clarified sample that has been subjected to at least one filtration step to remove host cells and other large impurities. The clarified sample can optionally also be pre-treated with a nuclease like Benzonase® (Merck KGaA, Germany).

Reducing or substantially removing divalent cations in the sample means that as many divalent cations as possible are removed from the sample or trapped or bound by physical or chemical means in the sample so that they are not available anymore for supporting binding of histidine residues to other histidine residues or to nucleic acids. In one embodiment at least the calcium ions are removed or trapped. In another embodiment at least the divalent cations are removed or trapped. In another embodiment the divalent and the trivalent ions are removed or trapped. The removal or trapping is preferably such that the sample is substantially free of divalent cations, especially of calcium ions. The more efficiently the removal or trapping of the divalent cations is done the more efficient is the reduction of aggregation. The divalent cations can be removed or trapped by any method that does not affect the viral particles. Suitable methods are the use of reverse osmosis, adsorption or chelation. Adsorption is typically done with suitable adsorbents, like solid phase adsorbents which are able to adsorb and thus trap the divalent cations. Examples of adsorbents are keratine based adsorbents, chemically modified polymeric membranes for metal ions removal or zeolithes.

Trapping of cations with a chelator or adsorbents means that the cations are bound by the chelator or adsorbent.

The chelator can be any chelator that is able to bind and thus trap divalent cations and optionally trivalent cations and that does not negatively interfere with the viral particles. Examples of suitable chelators are 8HQ (8-Hydroxyquinoline), EDTA (Ethylenediaminetetraacetic acid), EGTA (ethylene glycol tetraacetic acid), Nitrilotriacetic acid (NTA), iminodiacetic acid (IDA), DTPA Diethylenetriaminepentaacetic acid), HEDTA (N (Hydroxyethyl) ethylenediaminetriacetic acid), or mixtures thereof. Most preferred is EDTA.

The suitable concentration of the chelator in the sample depends on the amount of divalent cations that are present in the sample and on the amount of divalent cations that can be bound per chelator molecule. Typically, a final concentration in the sample of between 1 and 15 mM EDTA is suitable if cell lysis has been performed with Tween®20. The skilled person knows that if the sample comprises more or less divalent cations or if another chelator is used the amount of the chelator can be adjusted accordingly.

Typically, the amount of chelator is chosen such that in theory all divalent and trivalent cations present in the sample could be trapped.

Preferably, the pH of the sample for treatment with the chelator in step b) is adjusted to a pH between 6 and 9, preferably between 7 and 9. To achieve this, a suitable buffer can be added to the sample.

The temperature is not critical. The chelator can e.g. be added at room temperature or at any other temperature down to around 2 degrees Celsius.

Some chelators might be sensitive to light, like e.g. UV light. In such a case the treatment with the chelator is preferably done while the sample is shielded from exposure to light.

In another preferred embodiment the method further comprises treating the sample with a nuclease, whereby the nuclease can be added prior, in parallel or after the addition of the chelator. If the activity of the nuclease is dependent on the presence of certain divalent cations, the EDTA is preferably added after nuclease treatment.

Preferably, the sample is treated with the chelator for at least 30 seconds, preferably for an incubation time between 1 minute and 10 hours, most preferred between 1 minute and 10 minutes, whereby the sample can also be stored with the chelator for a longer period of time though for achieving the effect of reducing viral aggregation, assumably by disrupting the association between the viral particles and the process related impurities an incubation of typically 1 minute to 10 minutes is sufficient. The effect of chelation of the cations and thus reduction of aggregation can sometimes even be directly detected by a reduction of the turbidity of the sample.

After treatment with the chelator or equivalent removal or trapping of the divalent cations by other means, the sample is then subjected to at least one purification step to separate the viral particles from the process related impurities, especially from host cell proteins and/or host cell DNA. This purification can involve one or more filtration steps, one or more chromatographic purification steps or a step involving flocculation combined with filtration and/or centrifugation or any combination thereof. In a preferred embodiment it comprises at least one chromatographic step. Suitable filtration steps include tangential flow filtration and/or depth filtration. In one embodiment, the sample is filtered with a filter having a pore size between 0.1 and 0.8 $\mu$m.

Flocculants which are able to bind to nucleic acids and proteins are known to the skilled person. Examples are poly-l-amino acids, polymers or copolymers like polyacrylamide co-polymers and polyamines or chitosan. After incubation of the sample with the flocculant, the mixture is filtered and/or centrifuged to remove the flocculant as well as the impurities bound thereto.

In a very preferred embodiment, the viral particles are separated from the process-related impurities by optionally filtering the sample with a filter having a pore size between 0.1 and 0.8 $\mu$m and then subjecting the sample to affinity chromatography. While the viral particles are bound to the affinity matrix, the process-related impurities as well as if present the chelator flow through the column and are thus removed from the viral particles.

In one embodiment, the sample provided in step a) of the method of the present invention is the feed to be loaded on an affinity chromatography matrix.

Affinity chromatography may be performed by

a) loading the sample comprising the viral particles, if present the chelator and process related impurities onto an affinity matrix targeted against the viral particles under conditions that allow binding between the viral particles and the affinity resin;
b) undertaking at least one wash step; and
c) eluting the viral particles from the affinity matrix.

In a preferred embodiment, the affinity matrix has been equilibrated with a buffer comprising a chelator prior to addition of the sample to the affinity matrix. In one embodiment the equilibration buffer comprises 5 to 15 mM EDTA.

In another embodiment, the wash buffer used in step b) comprises a chelator. In one embodiment the wash buffer comprises 5 to 15 mM EDTA.

Suitable conditions and buffers for affinity chromatography are known to the skilled person. They might vary depending on the matrix that is used. Typically the pH is in the range between 6 and 9. PBS pH 7.0 to 7.5 is a good starting buffer. However, other standard neutral pH buffers such as 10-50 mM sodium phosphate or Tris can be used. Adding 0.1-0.2 M NaCl or KCl may prevent nonspecific adsorption due to protein/protein interactions.

In one embodiment, treatment with a chelator, preferably EDTA, is performed only with the wash buffer.

In another embodiment, another chromatographic method is used instead of or in combination with affinity chromatography. Suitable chromatographic methods are for example ion exchange chromatography or multimodal chromatography. In one embodiment, only a cation- or only an anion exchange or only a multimodal chromatography is performed. In another embodiment first an affinity chromatography and then at least one other chromatography selected from anion exchange, cation exchange or multimodal chromatography is performed.

In one embodiment, the sample is not treated with a chelator or any other method to remove or trap divalent cations prior to feeding the sample to a chromatography column. Treatment with the chelator takes place while the viral particles are bound to the chromatography matrix in a wash step by using a wash buffer comprising the chelator. In this case, step b) treatment of the sample with a chelator and step c) separation of the process related impurities from the viral particles are performed almost in parallel. As soon as the chelator present in the wash buffer traps the metal ions, aggregates are broken and the components of the aggregates which are not bound to the chromatography matrix are washed away and thus separated from the bound viral particles.

In a preferred embodiment the viral particles are AAV particles, like AAV2, AAV5, AAV 8 or AAV9 particles.

In another preferred embodiment, the affinity chromatography matrix is a membrane, most preferred a hydrogel membrane, with protein affinity ligands.

The viral particles resulting from the at least one purification step to remove process-related impurities show less aggregation compared to viral particles that have been treated identical but without removal or trapping of at least the divalent cations e.g. by treatment with and use of a chelator in any buffer.

The method of the present invention can thus be favorably used in any method for producing viral vectors. The adsorbens or the chelator can be added in any process step whereby care has to be taken that the process-related impurities are removed while the divalent and if required also the trivalent cations are still efficiently trapped or removed so that the host cell proteins and host cell nucleic acids that are still present in the sample are not attached to the viral particles and can efficiently be removed. The present invention is thus also directed to a process for producing viral particles comprising the steps of

a) Culturing cells to produce viral particles
b) Optionally lysing cells which comprise said viral particles
c) Obtaining a sample comprising the viral particles released from the cells
d) Removing or trapping at least divalent cations in the sample, preferably by treating the sample with a chelator
e) Performing at least one chromatographic purification

Preferably, the process comprises one or more additional steps selected from
Clarification

- filtration
- dialysis/ diafiltration
- tangential flow filtration
- treatment with nuclease, e.g. RNase and/or DNase
- treatment with chloroform
- ion exchange chromatography
- affinity chromatography
- multimodal chromatography
- hydrophobic interaction chromatography
- centrifugation
- PEG precipitation
- flocculation

The same conditions and embodiments that have been described for the method of the present invention also apply in the process for producing viral particles.

The present invention is also directed to a composition comprising viral particles, a pH buffer and a chelator. The pH of the composition is preferably between 7 and 9. The composition can comprise process-related impurities or it can be substantially free of process-related impurities. In any case the presence of the chelator ensures that if any process-related impurities, especially host cell DNA or fragments thereof do not aggregate with the viral particles and thus do not lead to aggregation of the viral particles.

In a preferred embodiment the chelator is EDTA.

It has been additionally found that treatment of the sample with a chelator or otherwise removing or trapping at least the divalent cations present in the sample provides several advantages in viral production and purification. It has been found that treatment of the sample with a chelator prior to an affinity chromatographic purification provides for reduced pressure increase during matrix loading and thus to higher loading capacity. The presence of aggregates in a sample often leads to column clogging. In addition, process-related impurities like host cell DNA and host cell proteins might interact especially with peptidic affinity ligands. Both effects can result in an increase in column pressure. It has been found that in cases where column loading is limited by increase in column pressure, the addition of a chelator to the sample to be fed to the column reduces column pressure and thus leads to higher loading capacity. The same effect can be obtained by pretreating the sample with an adsorbens.

Furthermore, the treatment of a sample with a chelator prior to feeding it on an affinity chromatography matrix also provides for improved purity of the viral vectors resulting from said affinity chromatography. It has been found that the presence of a chelator in the feed reduces co-elution of nucleic acid contaminants with the viral particles. Without wanting to be bound to theory, peptidic affinity ligands comprise histidine residues which might be able to enter into a metal cation mediated interaction with nucleic acids. This might lead to some part of the nucleic acids present in the sample not to flow through the matrix with other process related impurities but to stick to the matrix and be eluted together with the viral particles. As it is of high importance to remove all host cell DNA from the viral particles, a pre-treatment with a chelator prior to affinity chromatography as well as the use of a wash buffer comprising a chelator can reduce the amount of nucleic acid that is co-eluted with the viral particles. The resulting eluate of viral particles is substantially free of contaminating nucleic acids.

It has also been found that the process of the present invention provides for the separation of empty viral particles from full viral particles.

Aggregates, a product-related impurity of preparations of viral particles like AAV preparations, play a key role in the reduction of viral efficacy due to contributing to a reduction of viral transduction efficiency. Viral aggregation also increases the risk of immunogenicity. Viral aggregation can occur throughout the manufacturing process or in later stages such as formulation and storage. Nucleic acids were shown to mediate viral aggregation. This effect is typically reduced by nuclease digestion.

It has been found that increased transduction efficiency is shown when a chelator is used for chelation of divalent and if required also trivalent metal cations in the sample to be fed on the affinity matrix, followed by affinity purification. Preferably,1 to 15 mM of the chelator are also added to the equilibration buffer and/ the wash buffer. Compared to viral particles that have been purified identically beside the treatment with/addition of the chelator, the viral particles that have been treated with the chelator show significantly improved transduction efficiency.

Assumably, dissociation of complexes of viral particles with contaminating nucleic acids through removal of metal cations results in dissociation of viral aggregates and capsids free from contaminating nucleic acids on their surface. Both factors contribute to an increase in transduction efficiency as more viral attachment sites are available for attachment to the target cells. As a consequence, the present invention is also directed to a method for increasing the transduction efficiency of viral particles by

a) Providing a sample comprising viral particles and process-related impurities
b) Treating the sample with a chelator
c) Separating the viral particles from process related impurities, preferably, by affinity chromatography,

whereby the transduction efficiency of the viral particles is higher compared to the same amount of viral particles that have been otherwise obtained and treated identical beside the treatment with and presence of the chelator.

The present inventors have found a new way to reduce aggregation of viral particles and thus improve several parameters in manufacturing for clinical grade viral gene therapy vectors. The underlying effect seems to be the reduction of nucleic acid and potentially host cell protein binding through elimination of divalent and optionally also trivalent cations. While up to now one known way to reduce aggregate formation was to increase the salt concentration, it has now been found that eliminating certain ions, namely divalent cations, is possible without causing any negative side effects and reduces aggregate formation. Removal of divalent cations effectively leads to an interruption of the interaction between the viral particles and nucleic acids and proteins.

The present invention is especially favorable for AAV production. Current AAV-based gene therapies require large vector doses to secure efficacy in the clinic. Clinically effective doses are known to be able to elicit immune responses in patients leading to serious adverse events. One factor which can help to reduce vector dose and improve cost-efficiency of AAV manufacturing processes, is an increase in transduction efficiency. The present invention provides for this effect.

Another major challenge in AAV purification is achieving high titers of genome containing and clinically effective i.e. infectious AAV particles in short processing times. State-of-the art AAV purification is based on affinity resins which have a low productivity due to low flow rates.

Productivity increase can be achieved by coupling AAV-specific affinity ligands to a membrane or monolith support which allows for short processing times due to higher flow rates.

However, multiple factors contribute to a limitation of loading capacities. The present invention now provides a method to reduce column pressure during loading and to thus increase loading capacity.

Examples

**1. Material & Methods**

**Chromatography**

Material:

|  | Manufacturer | Cat. no. |
|---|---|---|
| Bis-Tris propane | Sigma-Aldrich | 394111 |
| EDTA | Sigma-Aldrich | A4892 |
| Glycine | Sigma-Aldrich | G7126 |
| PBS | Sigma-Aldrich | SAFSP4417-100TAB |
| Poloxamer 188 solution, 10% | Sigma-Aldrich | P5556 |
| Sodium chloride | Sigma-Aldrich | 106400 |

Buffer composition:

Equilibration buffer: PBS, 10 mM EDTA, pH 7.2-7.6

Sample: Benzonase-treated AAV2 lysate supplemented with 10 mM EDTA and sterile-filtered through a 0.22 $\mu$m PES filter, pH 7.2-8.0

Elution buffer: 100 mM glycine, 250 mM NaCl, 0.001% Poloxamer 188, pH 2.5

Eluate neutralization buffer: 550 mM Bis-Tris-Propane, 0.001% Poloxamer 188, pH 10.2

Device:

0.18 mL affinity membrane prototype targeting AAV2 capsids

Method:

| Step | Buffer/AAV sample | Flow rate (mL/min) | Volume (mL) | Neutralization buffer per 1 mL eluate fraction added prior to starting the run ($\mu$L) |
|---|---|---|---|---|
| Equilibration | Equilibration buffer | 1.44 | 15 | n/a |
| Sample application | EDTA-treated and sterile-filtered AAV2 lysate | 1.44 | until max. pressure drop | n/a |
| Wash | Equilibration buffer | 1.44 | 12 | n/a |
| 100% step elution | Elution buffer | 1.44 | 12 | 80 |

**AAV2 ELISA**

AAV2 ELISA (Progen, cat. no.: PRAAV2XP) was done according to the manufacturer's instructions.

**hcDNA quantification**

Host cell DNA analysis was done with the Quant-iT™ PicoGreen™ assay (Thermo Fisher; cat. no.: P11496) according to the manufacturer's instructions.

**DLS**

Material:

|  | **Manufacturer** | **Cat. no.** |
|---|---|---|
| Aurora 384 Well Plate | Waters Corporation | P8806-38403 |
| DynaPro Plate Reader III | Wyatt | - |
| Well Plate Sealing Tape | Waters Corporation | S8810-235307 |

Method:

Settings:

- Temperature: 20°C

- Acquisition time: 10 sec.

- Acquisitions: 30

- Measurements per well: 1

- Volume: 35 $\mu$L per well

Sample preparation:

- Thaw samples on ice

Analysis:

- Add buffer blank and samples to the 384 well plate

- Seal the plate and centrifuge at 2000 rpm, 1 min

- Measure the buffer to analyze matrix effects

- Measure the samples

- Intensity(%): kCnt/s; intensity at the current laser power and attenuation

- Mass(%): semiquantitative comparison between monomer and aggregate particle mass

**Transduction assay**

Material:

|  | Manufacturer | Cat. no. |
|---|---|---|
| Cytation 1 | Agilent | - |
| DMEM medium | Gibco | 61965-026 |
| Fetal Bovine Serum | Sigma Aldrich | F4135-500ML |
| HT1080 cells with fluorescent nucleus | Parental cell line from ATCC | - |
| Incubator | Thermo Fisher Scientific | - |
| Neubauer Chamber | NanoEntek | PK36.1 |
| PBS | Sigma-Aldrich | D8537-500ml |
| Trypsin-EDTA | Gibco | 25300-054 |
| 96 well plates, black, F bottom | Greiner | 655090 |
| 96 well plate | Corning | 500002008 |

Method:

Day 0

- Wash cells with PBS and detach them with trypsin-EDTA

- Count cells with a Neubauer chamber

- Dilution of cells to 50000 cells/mL in HT1080 complete media

- Fill outside wells with 0.1 mL of HT1080 complete media

- Add 0.1 mL/well in a 96-well plate (5000 cells/well)

- Once all the wells are filled, a gentle x-tilt back and forth 4-5 times followed by a similar gentle y-tilt helps to ensure an even distribution of cells

Day 1

- Prepare HT1080 complete media (15 mL/96 well plate, 10 mL for inner wells only)

- Thaw the AAV particles and hold on ice or at 4°C

- Dilute the AAV in complete media

- Aspirate the media of wells containing HT1080 cells

- Add 0.1 mL/well diluted AAV particles

- Determine the number of cells per well on day 1 of the assay by using the "initial cell count" value in the Cytation1 software Gen5

- Return the plate with the transduced cells to the virus incubator

Day 4

- Remove the media from the wells carefully and replace with 100 $\mu$L of PBS
- Image the plate on the Cytation1 collecting the cell count and the subpopulation of the fluorescent reporter expressed by the virus
- Determine the % GFP positive cells on the final day of the assay
- The following calculation will determine the titer of the virus:

$$\text{TU/mL} = (\% \text{ GFP positive} * \text{cells/well on day 1})/(\text{AAV dilution} * 0.1 \text{ mL})$$

NOTE: %GFP positive cells should be expressed as a decimal (where 60% is 0.60) when completing the calculation.

**2. Results**

2.1 Reduction of aggregates

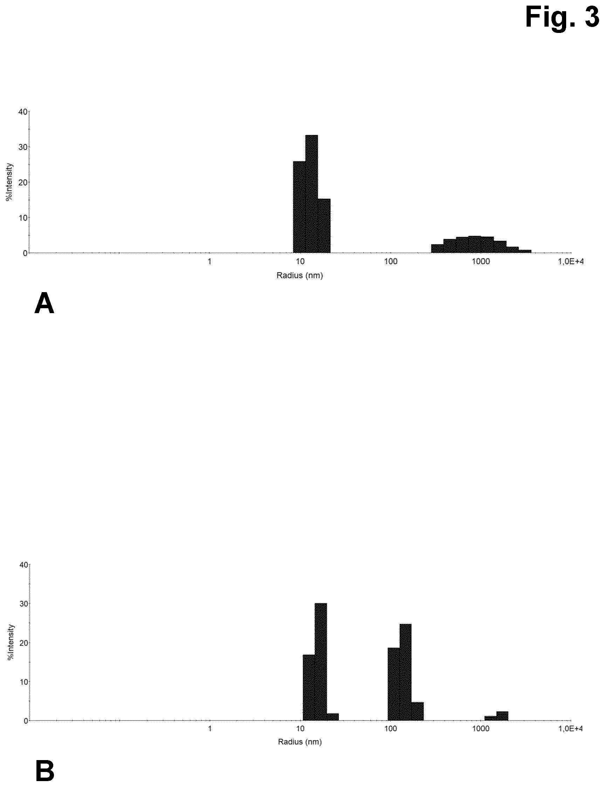

Fig. 3 shows a DLS analysis of elution pool fractions with and without EDTA pre-treatment
Fig. 3 A shows a chromatography eluate without EDTA pre-treatment.
Fig. 3 B shows a chromatography eluate with EDTA pre-treatment
DLS results confirmed that EDTA pre-treatment reduced the presence of large AAV2 aggregates in chromatography eluates.

**2.2 Effect of aggregate reduction on matrix loading**

**Table 1: Comparison of virus particle capacity and recovery of an AAV2-specific affinity membrane prototype with and without EDTA pre-treatment**

| Sample | Without ETDA pre-treatment | With EDTA pre-treatment |
|---|---|---|
| Load volume until max. pressure drop (mL) | 665 | 1362 |
| AAV2 loaded until max. pressure drop (vp/mL membrane) | 7.4E+14 | 2.0E+15 |
| AAV2 eluted until max. pressure drop (vp/mL membrane) | 6.8E+14 | 1.3E+15 |

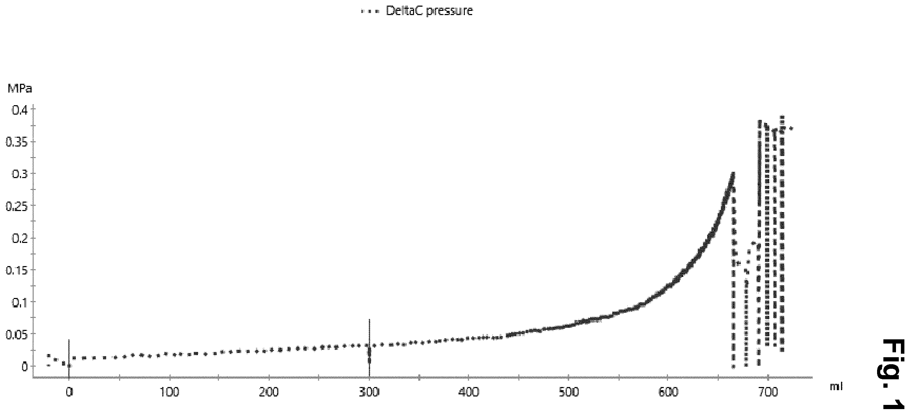

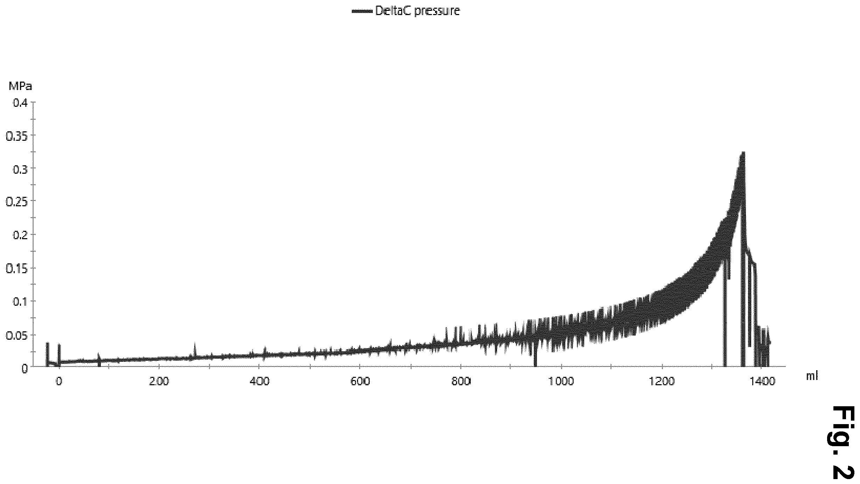

Aggregation of viral particles, and/or metal-ion mediated non-specific binding of host cell nucleic acids and proteins can cause a clogging of a filter or a chromatography matrix. By loading a membrane chromatography matrix with either a sample pretreated with the chelator EDTA and a sample that has not been pretreated with a chelator it could be shown that a treatment with a chelator reduces aggregation and non-specific interaction of host cell nucleic acids with the matrix and thus allows for higher loading of said matrix. Table 1 shows that EDTA pre-treatment enabled higher virus capacity and recovery with an AAV2-specific affinity membrane prototype.
The same effect can be seen from Figures 1 and 2.
Figures 1 and 2 show the pressure drop with and without EDTA pre-treatment. Fig. 1 shows the pressure curve without EDTA pre-treatment. Fig. 2 shows the pressure curve with EDTA pre-treatment.
Comparison of Figure 1 and 2 shows that EDTA pre-treatment allows for an increased loading volume before the maximum pressure drop occurs.

2.3 Effect on purity of viral particles

**Table 2: hcDNA analysis of flow-through and elution fractions of a chromatography run using AAV2-specific membrane affinity chromatography**

|  | Sample | hcDNA level (%) |
|---|---|---|
| Without EDTA pre-treatment | Flow-through pool | 96.8 |
|  | Elution pool | 0.0 |
| With EDTA pre-treatment | Flow-through pool | 100.4 |
|  | Elution pool | 0.0 |

A pretreatment according to the method of the present invention results in reduced aggregation and reduced binding of host cell DNA to viral particles.

Table 2 shows complete hcDNA recovery in the flow-through pool with EDTA pre-treatment. Potential trace contaminants of residual hcDNA in the elution pool could not be detected due to the limit of detection.

2.4 Effect on transduction efficiency of viral particles

**Table 3: Comparison of the number of total AAV2 capsids and AAV2 transducing units of an elution pool without EDTA pre-treatment and an elution pool with EDTA pre-treatment**

|  | Total AAV particles (vp) | Functional AAV particles (TU) | Increase in total AAV particles in relation to EDTA-free experiment | Increase in functional AAV particles in relation to EDTA-free experiment |
|---|---|---|---|---|
| Elution pool without EDTA pre-treatment | 1.2E+14 | 8.0E+08 | n/a | n/a |
| Elution pool with EDTA pre-treatment | 2.0E+14 | 1.7E+09 | 59.9% | 114.9% |

AAV2 ELISA was used to determine the number of total AAV2 capsids in both elution pools. The EDTA pre-treated pool showed a ~ 60% higher number of total capsids. A transduction assay with HT1080 cells confirmed that the number of transducing units was not only increased by 60% but by 115% (Tab.3). That clearly shows that the increase in transduction efficiency is not only due to the overall higher number of viral particles in the sample treated with EDTA but also due to the treatment with EDTA.

**Claims**

1.   A method for reducing aggregation of viral particles by

   a. Providing a sample comprising viral particles and process related impurities
   b. reducing the amount of divalent cations in the sample
   c. Separating the viral particles from process related impurities

2.   Method according to claim 1, **characterized in that** a chelator is added in step b) that is able to bind at least divalent cations.

3.   Method according to claims 1 or 2, **characterized in that** the amount of chelator added in step b) is such that it is sufficient to theoretically bind all divalent cations present in the sample.

4.   Method according to one or more of claims 1 to 3, **characterized in that** the chelator is EDTA.

5.   Method according to one or more of claims 1 to 4, **characterized in that** step b) is performed for at least 30 seconds.

6.   Method according to one or more of claims 1 to 5, **characterized in that** the viral particles are AAV particles.

7. Method according to one or more of claims 1 to 6, **characterized in that** the sample is a clarified sample.

8. Method according to one or more of claims 1 to 7, **characterized in that** step c) comprises an affinity chromatography step.

9. Method according to one or more of claims 1 to 8, **characterized in that** step c) comprises first filtering the sample of step b) and then feeding the sample to an affinity chromatography matrix.

10. Process for producing viral particles comprising the steps of

    a) Culturing cells to produce viral particles
    b) Optionally lysing cells which comprise said viral particles
    c) Obtaining a sample comprising the viral particles released from the cells
    d) Removing or trapping at least divalent cations present in the sample
    e) Performing at least one chromatographic purification

11. Process according to claim 10, **characterized in that** a chelator is added in step d) that is able to bind at least divalent cations.

12. Process according to claims 10 or 11, **characterized in that** the amount of chelator added in step d) is such that it is sufficient to theoretically bind all divalent cations present in the sample.

13. Process according to one or more of claims 10 to 12, **characterized in that** the chromatographic purification in step e) is affinity chromatography.

14. Process according to claims one or more of claims 10 to 13, **characterized in that** the chelator EDTA is added in step d).

15. Process according to one or more of claims 10 to 14, **characterized in that** the viral particles are AAV particles.

16. Process according to one or more of claims 10 to 15, **characterized in that** it comprises one or more of the following steps:

    - clarification
    - filtration
    - dialysis/ diafiltration
    - tangential flow filtration
    - treatment with nuclease
    - treatment with chloroform
    - ion exchange chromatography
    - multimodal chromatography
    - affinity chromatography
    - hydrophobic interaction chromatography
    - centrifugation
    - PEG precipitation
    - flocculation

17. Use of the method according to one or more of claims 1 to 9 for increasing purity and/or transduction efficiency of viral particles.

18. A composition comprising viral particles, a pH buffer and a chelator.

19. A composition according to claim 14, **characterized in that** the chelator is EDTA.

# Fig. 1

Fig. 2

# Fig. 3

A

B

**EP 4 606 892 A1**

<table>
<tr><td colspan="5" align="center">**EUROPEAN SEARCH REPORT**</td><td>**Application Number**<br>EP 24 15 9462</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | JP 2006 204201 A (NIPPON SEKIJUJISHA)<br>10 August 2006 (2006-08-10)<br>* paragraphs [0009], [0014], [0016], [0025] - [0026], [0029] - [0031], [0037] * | 1-6,17, 18<br><br>8,9 | INV.<br>C12N7/02<br>C12N15/86<br>C12N15/864 |
| X | EP 1 636 352 B1 (MICROBIX BIOSYSTEMS INC [CA]) 18 August 2010 (2010-08-18)<br>* paragraphs [0081], [0030], [0095], [0082] * | 1-5,7, 17,18 | |
| Y | US 2021/370199 A1 (ZHANG CLAIRE G [US] ET AL) 2 December 2021 (2021-12-02)<br>* paragraphs [0007], [0285] * | 8,9,13 | |
| X<br><br>Y | US 2022/325250 A1 (REINAUER EVA [DE] ET AL) 13 October 2022 (2022-10-13)<br>* paragraphs [0001], [0004], [0017], [0044], [0048], [0065], [0113] * | 10-12, 14-19<br><br>13 | |
| X | US 10 626 376 B2 (ST JUDE CHILDRENS RES HOSPITAL [US]) 21 April 2020 (2020-04-21)<br>* example 1 * | 18 | **TECHNICAL FIELDS SEARCHED (IPC)**<br><br>C12N<br>C07K |
| Y | EL ANDARI JIHAD ET AL: "Production, Processing, and Characterization of Synthetic AAV Gene Therapy Vectors", BIOTECHNOLOGY JOURNAL,<br>vol. 16, no. 1,<br>7 October 2020 (2020-10-07), page 2000025, XP055791742,<br>DE<br>ISSN: 1860-6768, DOI: 10.1002/biot.202000025<br>Retrieved from the Internet: URL:https://onlinelibrary.wiley.com/doi/full-xml/10.1002/biot.202000025><br>* the whole document * | 8,9,13 | |

The present search report has been drawn up for all claims

| Place of search | Date of completion of the search | Examiner |
|---|---|---|
| Munich | 11 June 2024 | Dogrammatzis, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 24 15 9462

This annex lists the patent family members relating to the patent documents cited in the above-mentioned European search report.
The members are as contained in the European Patent Office EDP file on
The European Patent Office is in no way liable for these particulars which are merely given for the purpose of information.

11-06-2024

| Patent document cited in search report | | Publication date | Patent family member(s) | | Publication date |
|---|---|---|---|---|---|
| JP 2006204201 A | | 10-08-2006 | NONE | | |
| EP 1636352 B1 | | 18-08-2010 | AR | 044843 A1 | 05-10-2005 |
| | | | AT | E478137 T1 | 15-09-2010 |
| | | | AU | 2004249802 A1 | 29-12-2004 |
| | | | CA | 2503774 A1 | 29-12-2004 |
| | | | EP | 1636352 A1 | 22-03-2006 |
| | | | JP | 4558652 B2 | 06-10-2010 |
| | | | JP | 2006511240 A | 06-04-2006 |
| | | | MX | PA05005306 A | 26-10-2005 |
| | | | US | 2005186223 A1 | 25-08-2005 |
| | | | US | 2009035837 A1 | 05-02-2009 |
| | | | WO | 2004113518 A1 | 29-12-2004 |
| US 2021370199 A1 | | 02-12-2021 | AU | 2019285186 A1 | 07-01-2021 |
| | | | CA | 3102817 A1 | 19-12-2019 |
| | | | CN | 112469822 A | 09-03-2021 |
| | | | EP | 3807405 A2 | 21-04-2021 |
| | | | IL | 279193 A | 31-01-2021 |
| | | | JP | 7385603 B2 | 22-11-2023 |
| | | | JP | 2021526829 A | 11-10-2021 |
| | | | KR | 20210020100 A | 23-02-2021 |
| | | | US | 2021370199 A1 | 02-12-2021 |
| | | | WO | 2019241535 A2 | 19-12-2019 |
| US 2022325250 A1 | | 13-10-2022 | EP | 4013436 A1 | 22-06-2022 |
| | | | US | 2022325250 A1 | 13-10-2022 |
| | | | WO | 2021028559 A1 | 18-02-2021 |
| US 10626376 B2 | | 21-04-2020 | US | 2018135024 A1 | 17-05-2018 |
| | | | WO | 2018089192 A1 | 17-05-2018 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005118792 A
- CN 113980917
- WO 04073843 A
- WO 2010027955 A
- WO 2014018635 A

**Non-patent literature cited in the description**

- **COFFIN et al.** Retroviruses. Cold Spring Harbor Laboratory Press, 1997, 758-763
- **MUZYCZKA**. *Curr. Top. Micro. Immunol.*, 1992, vol. 158, 97-129
- **BUFFERS**. A Guide for the Preparation and Use of Buffers in Biological Systems. Calbiochem Corporation, 1975